(19) Europäisches Patentamt
European Patent Office
Office européen des brevets

(11) Publication number: **0 502 498 A1**

(12) **EUROPEAN PATENT APPLICATION**

(21) Application number: **92103682.8**

(22) Date of filing: **04.03.92**

(51) Int. Cl.[5]: **C07C 311/17**, C07C 311/13, C07C 311/29, C07C 311/46, C07C 311/04, C07D 213/71, C07D 333/34, A61K 31/19, A61K 31/38, A61K 31/44

(30) Priority: **07.03.91 JP 125597/91**

(43) Date of publication of application: **09.09.92 Bulletin 92/37**

(84) Designated Contracting States: **DE FR GB IT**

(71) Applicant: **TANABE SEIYAKU CO., LTD.**
**2-10, Dosho-machi 3-chome**
**Chuo-ku Osaka(JP)**

(72) Inventor: **Iijima, Ikuo**
**No. 1-1-8-403, Ohara 7-chome**
**Urawa-shi, Saitama-ken(JP)**
Inventor: **Yamashita, Toyoharu**
**No. 414-30, Oazamukoyama**
**Ageo-shi, Saitama-ken(JP)**
Inventor: **Okumura, Kunihito**
**No. 12-23, Kamikizaki 6-chome**
**Urawa-shi, Saitama-ken(JP)**
Inventor: **Inamasu, Masanori**
**No. 4-3-407, Waseda 3-chome**
**Misato-shi, Saitama-ken(JP)**
Inventor: **Ohtani, Akio**
**No. 6-35-519, Namiki 1-chome**
**Kawaguchi-shi, Saitama-ken(JP)**

(74) Representative: **Hansen, Bernd, Dr.**
**Dipl.-Chem. et al**
**Hoffmann, Eitle & Partner Patent- und Rechtsanwälte Arabellastrasse 4 Postfach 81 04 20**
**W-8000 München 81(DE)**

(54) **Phenoxyalkanoic acid derivatives and process for preparation thereof.**

(57) There is disclosed a phenoxyalkanoic acid compound of the formula:

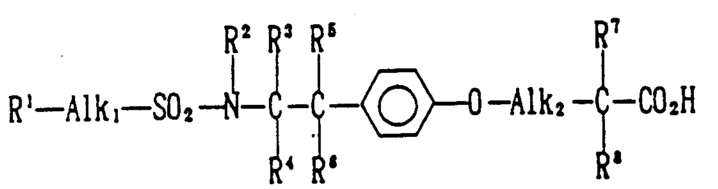

(I)

wherein $R^1$ is a substituted or unsubstituted phenyl group, naphthyl group, a sulfur- or nitrogen-containing 5- or 6-membered heterocyclic group or a lower alkyl group; $R^2$ is hydrogen atom or a lower alkyl group; one or two of $R^3$ - $R^6$ is (are) lower alkyl group(s) and others are hydrogen atoms; each of $R^7$ and $R^8$ is a lower alkyl group; and each of $Alk_1$ and $Alk_2$ is single bond or a lower alkylene group, or a pharmaceutically acceptable amide or salt thereof, which have excellent hypolipidemic activities and are useful for prophylaxis and treatment of hyperlipidemia.

EP 0 502 498 A1

This invention relates to a phenoxyalkanoic acid derivative useful as a hyperlipidemic agent and processes for preparation thereof.

## BACKGROUND OF THE INVENTION

Hyperlipidemia such as hypercholesterolemia has been known to be a major risk factor for arteriosclerosis, including atherosclerosis, Mönckeberg arteriosclerosis and the like, and drugs such as Bezafibrate [chemical name : 2-[4-{2-(p-chlorobenzamino) ethyl}phenoxy]-2-methylpropionic acid ] have been clinically used for prophylaxis and treatment of hyperlipidemia.

On the other hand, Japanese Patent Publication (unexamined) No. 62 (1989) discloses a group of compounds having a lower alkyl group on the ethyl moiety of 4-{2-(benzenesulfonylamino)ethyl} phenoxyacetic acid which have platelet-aggregation inhibitory activities, but no hypolipidemic activity of said compounds has been known up to now.

Alternatively, Japanese Patent Publication (unexamined) No. 122250 (1979) discloses a group of compounds having a lower alkyl group on the acetic acid moiety of 4-{2-(benzenesulfonylamino)-ethyl}phenoxyacetic acid which have platelet-aggregation inhibitory activities and hypolipidemic activities. However, the hypolipidemic activities of said compounds are still unsatisfactory for clinical use.

## SUMMARY OF THE INVENTION

As a result of various investigation, the inventors of the present invention have found that a novel pharmaceutical compound having potent hypolipidemic activities, especially excellent hypocholesterolemic activities is obtained by introducing lower alkyl groups to both of ethyl and acetic acid moieties of 4-{2-(benzenesulfonylamino)ethyl}phenoxyacetic acid and the like.

Thus, an object of the present invention is to provide a novel phenoxyalkanoic acid derivative which is useful for prophylaxis and therapeutic treatment of hyperlipidemia and/or arteriosclerosis. Another object is to provide a novel pharmaceutical composition useful as a hypolipidemic agent, which contains said phenoxyalkanoic acid derivative as the therapeutically active ingredient. And still another object is to provide processes for preparing said novel phenoxyalkanoic acid derivative. These and other objects or advantages of the invention will be apparent to those skilled in the art from the following description.

## DETAILED DESCRIPTION OF THE INVENTION

This invention relates to a phenoxyalkanoic acid derivative of the following formula [I] :

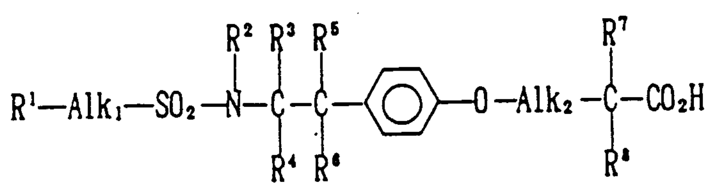

[I]

wherein $R^1$ is a substituted or unsubstituted phenyl group, naphthyl group, a sulfur- or nitrogen-containing 5- or 6-membered heterocyclic group or a lower alkyl group; $R^2$ is hydrogen atom or a lower alkyl group; one or two of $R^3$ - $R^6$ is (are) lower alkyl group(s) and others are hydrogen atoms; each of $R^7$ and $R^8$ is a lower alkyl group; and each of $Alk_1$ and $Alk_2$ is single bond or a lower alkylene group and a pharmaceutically acceptable amide or salt thereof.

Compared with the known compounds having similar chemical structure, the phenoxyalkanoic acid derivative [I] has much more excellent hypolipidemic activities and is very useful for the prophylaxis and therapeutic treatment of hyperlipidemia.

For example, when the decreasing effect on the serum total cholesterol level was examined by using rats fed with a diet supplemented by cholesterol and sodium cholate, sodium 2-[4-{(RS)-2-(p-chlorobenzenesulfonylamino)propyl}phenoxy]-2-methylpropionate of the present invention showed about more than 3 times stronger decreasing effect than sodium 2-[4-{2-(benzenesulfonylamino)ethyl}phenoxy]-2-methylpropionate of the Japanese Patent Publication (unexamined) No. 122250 (1979).

Examples of the phenoxyalkanoic acid derivative of the present invention include those of the formula [I] in which $R^1$ is phenyl group, a phenyl group substituted by a group or atom selected from the group

consisting of a halogen atom, a lower alkyl group, a lower alkoxy group and a lower alkanoylamino group, naphthyl group, a sulfur- or nitrogen-containing 5- or 6-membered heterocyclic group such as thienyl group or pyridyl group or a lower alkyl group.

Among these derivatives, pharmaceutically preferred examples of the compounds are those of the formula [I] wherein $R^1$ is phenyl group, a halogenophenyl group, a lower alkylphenyl group, a lower alkoxyphenyl group or thienyl group; and each of $Alkl_1$ and $Alk_2$ is single bond.

Pharmaceutically more preferred examples of the compounds are those of the formula [I] wherein $R^1$ is a halogenophenyl group, a lower alkylphenyl group or thienyl group; each of $R^2$ and $R^4$-$R^6$ is hydrogen atom; each of $R^3$, $R^7$ and $R^8$ is a lower alkyl group; and each of $Alk_1$ and $Alk_2$ is single bond.

The compound [I] of the present invention may be used for pharmaceutical application either in the free form or a pharmaceutically acceptable amide or salt. Examples of the pharmaceutically acceptable amides include unsubstituted amide and mono- or di-lower alkyl amides which may have carboxyl group on the alkyl moiety thereof, such as a lower alkylamide, a di-lower alkylamide, a carboxy-lower alkylamide and a di(carboxy-lower alkyl)amide, and the like. Examples of the pharmaceutically acceptable salts include inorganic or organic salts such as alkali metal salts (e.g., sodium salt, potassium salt), alkaline earth metal salts (eg., calcium salt, magnesium salt), heavy metal salts (e.g., zinc salt), ammonium salt, organic amine salts (e.g., triethylamine salt, pyridine salt, ethanolamine salt, a basic amino acid salt), and the like.

In the above-mentioned examples of the compound of the formula [I] or a pharmaceutically acceptable amide thereof, the lower alkyl group, the lower alkoxyl group , the lower alkanoylamino group and the lower alkylene group mean an alkyl group, an alkoxyl group, an alkanoylamino group and an alkylene group of 1 to 6 carbon atoms, preferably 1 to 4 carbon atoms, respectively.

The phenoxyalkanoic acid derivative [I] of the present invention may exist in the form of 2 to 8 optically active isomers when the geminal substituents, i.e., $R^3$ & $R^4$, $R^5$ & $R^6$ and/or $R^7$ & $R^8$ of the formula [I] are different from each other, and this invention includes all of these optically active isomers and a mixture thereof.

The compound [I], a pharmaceutically acceptable amide or salt thereof may be administered either orally or parenterally to a warm-blooded animal, including human being and may also be used in the form of a pharmaceutical preparation containing the same compound in admixture with pharmaceutical excipients suitable for oral or parenteral administration (such as conventional pharmaceutically acceptable carriers or diluents). The pharmaceutical preparations may be in solid form such as tablets, capsules and powders, or in liquid form such as solutions, suspensions or emulsions. Further, when administered parenterally, it may be used in the form of injections.

The daily dose of the compound [I] of the present invention may vary depending on administration route, age, weight, conditions of the patient, and kinds of the diseases to be cured, but it is usually in the range of about 0.1 to 100 mg/kg of body weight, preferably about 0.5 to 10 mg/kg of body weight.

As mentioned hereinbefore, the phenoxyalkanoic acid derivative [I], a pharmaceutically acceptable amide and salt thereof have potent hypolipidemic activities. Especially, the present compound [I] of the present invention is characterized in that it can decrease serum total cholesterol level. Therefore, the compound of the invention is useful for treatment or prophylaxis of hyperlipidemia (e. g., hypercholesterolemia) or arteriosclerosis (e. g., atherosclerosis, Mönckeberg arteriosclerosis) in a warm-blooded animal including human being.

According to this invention, the compound [I] may be prepared by

a) condensing an amine compound of the formula [II] :

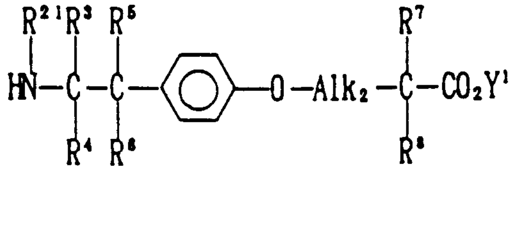

[ I I]

wherein $R^{21}$ is hydrogen atom or a lower alkyl group; $-CO_2Y^1$ is carboxyl group or a protected carboxyl group; and other symbols are the same as defined above, or a salt thereof with a sulfonic acid compound of the formula [III]:

$R^1$-$Alk_1$-$SO_2$ -$X^1$ [III]

wherein $X^1$ is a reactive residue or hydroxy group and other symbols are the same as defined above, or a salt thereof; or

b) condensing a phenol compound of the formula [IV]:

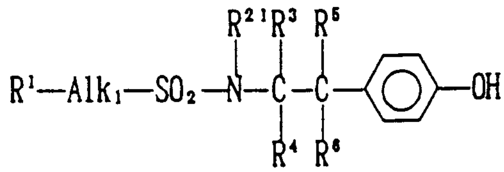

[IV]

wherein symbols are the same as defined above, or a salt thereof with an acetic acid derivative of the formula [V]:

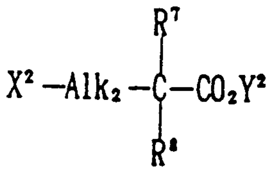

[V]

wherein $X^2$ is a reactive residue or hydroxy group, $-CO_2Y^2$ is carboxyl group or a protected carboxyl group and other symbols are the same as defined above, or a salt thereof;

(c) when $-CO_2Y^1$ or $-CO_2Y^2$ is a protected carboxyl group, removing the protecting group therefrom; and

(d) when $R^{21}$ is hydrogen atom, optionally subjecting the product to N-alkylation.

In the above-mentioned starting compounds [II] and [V], conventional protecting groups which are easily removed according to the conventional method such as hydrolysis, acid-treatment, reduction and the like may be used as the protecting groups of the carboxyl group ($Y^1$ and $Y^2$). Examples of the protecting groups include a lower alkyl group, benzyl group, a lower alkoxybenzyl group, a nitrobenzyl group, benzhydryl group and the like. And a halogen atom, a lower alkylsulfonyloxy group, a benzenesulfonyloxy group which may be substituted by a lower alkyl group, and the like may be used as the reactive residue ($X^1$ and $X^2$).

The condensation reactions of the amine compound [II] with the sulfonic acid compound [III] and the phenol compound [IV] with the acetic acid derivative [V] may be carried out in the presence of an acid acceptor. The acid acceptor includes conventional acid acceptors and preferred examples thereof include an alkali metal hydroxide, an alkali metal bicarbonate, an alkali metal carbonate, an alkaline earth metal carbonate, organic bases such as pyridine, trimethylamine and triethylamine.

The amine compound [II] and the acetic acid derivative [V] in which $-CO_2Y^1$ and $-CO_2Y^2$ thereof are free carboxyl group, the sulfonic acid compound [III] in which $X^1$ is hydroxy group and the phenol compound [IV] may be subjected to the condensation reaction in the form of a salt thereof. Examples of the salts include an alkali metal salt, an alkaline earth metal salt and the like. These reactions are preferably carried out in or without a solvent. Water, a lower alkanol, acetone, ethyl acetate, ether, benzene, tetrahydrofuran, methylenechloride, chloroform, dioxane and the like are suitably used as the solvent. The condensation reaction of the amine compound [II] with the sulfonic acid compound [III] may be carried out under cooling or with or without heating, preferably at a temperature of 0 °C to 30 °C. The condensation reaction of the phenol compound [IV] with the acetic acid derivative [V] may be carried out at room temperature or with heating, preferably at a temperature of 40 °C to 100 °C.

When either of $-CO_2Y^1$ and $-CO_2Y^2$ in the resulting product is a protected carboxyl group, the protecting group may be removed in a conventional manner, for example, by hydrolysis, acid-treatment, reduction and the like. A method for removal of the protecting group may be selected according to the protecting group to be removed.

Moreover, when $R^{21}$ of the product is hydrogen atom, the product may be subjected to N-alkylation. The N-alkylation can be carried out by reacting the product with a lower alkyl halide in the presence or absence of an acid acceptor. Examples of the acid acceptor include an alkali metal hydroxide, an alkali metal hydride, an alkali metal carbonate and a mixture of an alkali metal halide and aluminum oxide. The reaction may be carried out in a suitable solvent under ice-cooling or with heating, preferably at a temperature of 20 °C to 70 °C. Examples of the solvent include acetone, ethyl acetate, dimethylformamide, dimethylsulfoxide, acetonitrile, tetrahydrofuran, dioxane and the like.

Further, the thus-obtained phenoxyalkanoic acid derivative [I] may be converted to the amide thereof in a conventional manner.

Such conversion may be carried out, for example, by reacting the compound [I] or a reactive derivative thereof (e. g., acid halide, activated ester) with ammonia or a mono- or di-lower alkylamine which may have carboxyl group on the alkyl moiety thereof.

The amide formation reaction may be carried out by conventional manners, for example, in the presence or absence of an acid acceptor in or without a solvent. Examples of the acid acceptor include an alkali metal hydroxide, an alkali metal bicarbonate, an alkali metal carbonate, an alkaline earth metal carbonate, organic bases such as pyridine, trimethylamine, triethylamine. Chloroform, methylenechloride, diethylether, tetrahydrofuran and dimethylformamide are preferably used as the solvent. The reaction is carried out under ice-cooling or at a temperature of 0 °C to 70 °C, preferably at a temperature of 0 °C to 20 °C.

Concomitantly, the starting compound [II] of the present invention is novel and can be prepared, for example, by condensing an aminophenol compound of the formula [VI] :

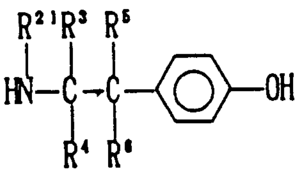

[VI]

wherein symbols are the same as defined above, or a salt thereof with the acetic acid derivative [V] or a salt thereof in a suitable solvent in the presence of an acid acceptor.

On the other hand, the starting compound [IV] can be prepared, for example, by condensing an alkylamine compound or a salt thereof of the formula [VII] :

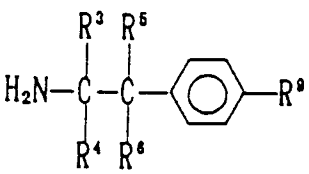

[VII]

wherein $R^9$ is hydroxy group or a protected hydroxy group and other symbols are the same as defined above, or a salt thereof with the sulfonic acid compound [III] or a salt thereof in a suitable solvent in the presence of an acid acceptor, and when $R^9$ is a protected hydroxy group, removing the protecting group therefrom and optionally subjecting the product to N-alkylation.

## Experiment

(Effect on serum total cholesterol level)

Male SD rats (body weight : 120 to 140 g, one group consisting of 5 rats) were fed ad libitum for 4 days with a hypercholesterolemic diet (CE-2 diet containing 2 w/w % of cholesterol and 0.5 w/w % of sodium cholate). Then, the rats were further fed ad libitum with the hypercholesterolemic diet containing 3 mg% or 10 mg% of a test compound. On the other hand, control group of rats were further fed with the diet without the test compound. Three days later, the rats were anesthetized with ether. After the body weight of the rats were measured, blood was collected from abdominal aorta thereof. The blood was allowed to stand at room temperature for one hour and centrifuged to obtain serum. Then, the total cholesterol level in the serum was measured enzymatically according to the method described in Clinical Chemistry, vol. 20, page 470 (1974).

On the basis of the results obtained above, the effect of the test compound on the serum total cholesterol level was estimated according to the following formula:
Percentage decrease in serum total cholesterol level

$$= \left[ 1 - \frac{\text{Average value of serum total cholesterol levels in the medicated group}}{\text{Average value of serum total cholesterol levels in the control group}^{*}} \right] \times 100$$

* : average value of serum total cholesterol levels in the control group was 168 - 231 mg/dl

Results are shown in following Table 1.

Table 1

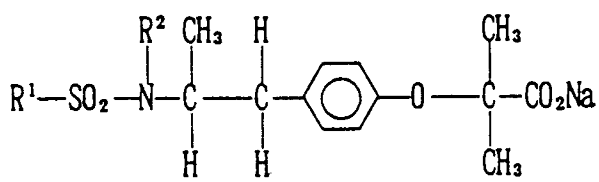

| Test Compound Nos. | $R^1$ | $R^2$ | Dose (mg%) | Percentage Decrease in STC* |
|---|---|---|---|---|
| 1 | 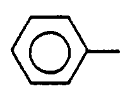 | H | 10 | 73 |
| 2 | 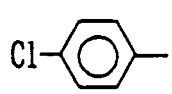 | H | 3 | 58 |
| 3 | 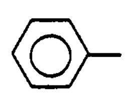 | $CH_3$ | 10 | 63 |
| 4 | 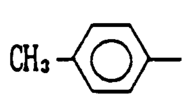 | H | 3 | 44 |
| 5 | 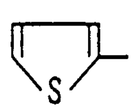 | H | 3 | 49 |

* : STC=Serum total cholesterol level

Further, when each of the compounds listed in Table 1 was orally administered to male ddY mice at a

dose of 1000 mg/kg, no mouse died 72 hours after the administration thereof.

Example 1

(1) 2.6 g of 4-{(RS)-2-(benzenesulfonylamino)propyl}phenol, 1.38 g of potassium carbonate and 1.95 g of ethyl 2-bromo-2-methylpropionate are added to 30 ml of acetone and the mixture is refluxed overnight. Further, 1.38 g of potassium carbonate and 1.95 g of ethyl 2-bromo-2-methylpropionate are added thereto, and the mixture is refluxed overnight. Inorganic substance is removed from the reaction mixture by filtration, and the filtrate is condensed. The residue is dissolved in ethyl acetate, washed with water, dried, and then the solvent is evaporated therefrom to obtain 3.3 g of crude ethyl 2-[4-{(RS)-2-(benzenesulfonylamino) propyl}phenoxy]-2-methylpropionate.
(2) The above-obtained product is dissolved in 30 ml of methanol and 20 ml of 10% aqueous potassium hydroxide solution are added thereto. The mixture is stirred at room temperature for 2 hours. The reaction mixture is condensed and acidified with conc. hydrochloric acid and extracted with ethyl acetate. The ethyl acetate layer is extracted with 5% aqueous sodium bicarbonate solution. The extract is acidified with conc. hydrochloric acid, and further extracted with chloroform. The extract is dried and evaporated to remove the solvent to obtain 2.35 g of 2-[4-{(RS)-2-(benzenesulfonylamino)-propyl}phenoxy]-2-methylpropionic acid as a pale yellow oil. Yield : 70%
IR (Nujol) $cm^{-1}$ : 1770(sh.), 1718
Mass (m/z) : 377 ($M^+$)
NMR ($CDCl_3$) $\delta$ : 1.06 (d., J = 6.4Hz, 3H), 1.60(s., 6H)
(3) 5.66 g of the above-obtained product is added to 17 ml of 1N aqueous sodium hydroxide solution and purified by column chromatography filled with a non-ionic absorbance resin (trade name : DIAION SP207, manufactured by MITSUBISHI KASEI CORPORATION, solvent : water and 50% methanol). The fractions containing the product are collected and are lyophilized to remove the solvent to obtain 5.09 g of sodium 2-[4-{(RS)-2-(benzenesulfonylamino)propyl}phenoxy]-2-methylpropionate as colorless powder.
Yield : 85%
IR (Nujol) $cm^{-1}$ : 1590
Mass (m/z) : 422 $(M+Na)^+$, 400 $(M+H)^+$
NMR ($D_2O$) $\delta$ : 1.01 (d., J = 6.4Hz, 3H), 1.52(s., 6H)

Example 2

(1) 2.60 g of 4-{(RS)-2-(benzenesulfonylamino)propyl}phenol, 1.84 g of methyl 5-bromo-2,2-dimethylpentanoate and 3.10 g of potassium carbonate are added to 50 ml of acetone and the mixture is stirred at room temperature overnight. Further, the mixture is refluxed for 24 hours. Inorganic substance is removed from the reaction mixture by filtration, and the filtrate is condensed. The residue is dissolved in ethyl acetate, washed with water, dried, and then the solvent is evaporated therefrom. The residue is separated and purified by silica gel column chromatography (solvent: ethyl acetate / n-hexane = 3 : 7) to obtain 2.02 g of methyl 5-[4-{(RS)-2-(benzenesulfonylamino) propyl}phenoxy]-2,2-dimethylpentanoate as an oil.
Yield : 62%
IR ( Liquid) $cm^{-1}$ : 1730
Mass (m/z) : 433 ($M^+$)
NMR ($CDCl_3$) $\delta$ : 1.08 (d., J = 6.4Hz, 3H), 1.22(s., 6H), 3.66(s., 3H)
(2) 1.98 g of the above-obtained product is dissolved in 30 ml of methanol and 4 ml of 4N aqueous sodium hydroxide solution are added thereto, and the mixture is refluxed for 5 hours and evaporated to remove the solvent. The residue is dissolved in water and acidified with 10% hydrochloric acid . The solution is extracted with ethyl acetate and the extract is washed with water, dried and evaporated to remove the solvent.

The residue is separated and purified by silica gel column chromatography (solvent : chloroform/ ethyl acetate = 7 : 3) to obtain 1.57 g of 5-[4-{(RS)-2-(benzenesulfonylamino)propyl} phenoxy]-2,2-dimethylpentanoic acid as an oil.
Yield : 82%
IR (Liquid) $cm^{-1}$ : 1740 (sh.), 1700
Mass (m/z) : 419 ($M^+$)
NMR ($CDCl_3$) $\delta$ : 1.09 (d., J = 6.4Hz, 3H), 1.26(s., 6H)
(3) The above-obtained product is treated in the same manner as described in Example 1-(3) and the

resulting product is recrystallized from isopropyl alcohol to obtain 1.0 g of sodium 5-[4-{(RS)-2-(benzenesulfonylamino)propyl}phenoxy]-2,2-dimethylpentanoate. Yield : 61%
M.P. : 135-136 °C
IR (Nujol) $cm^{-1}$ : 1540
Mass (m/z) : 464 $(M+Na)^+$, 442 $(M+H)^+$
NMR ($D_2O$) δ : 1.13 (s., 6H), 1.17(d., J=6.6Hz, 3H)

Example 3

(1) 2.0 g of ethyl 2-[4-{(RS)-2-aminopropyl}phenoxy]-2-methylpropionate oxalate and 8.0 g of potassium carbonate are added to a mixture of 20 ml of ethyl acetate and 10 ml of water. 1.17 g of p-chlorobenzenesulfonylchloride are added thereto and the mixture is stirred at room temperature for one hour. The ethyl acetate layer is separated from the reaction mixture, washed, dried and evaporated to remove the solvent. The residue is recrystallized from a mixture of ethyl acetate and n-hexane to obtain 2.2 g of ethyl 2-[4-{(RS)-2-(p-chlorobenzenesulfonylamino) propyl}phenoxy] -2-methylpropionate as colorless prisms. M. P. : 78-80 °C
(2) 15.0 g of the above-obtained product are dissolved in 120 ml of ethanol, and 40 ml of 3N aqueous sodium hydroxide solution are added thereto and the mixture is stirred at room temperature for 2 hours. Ethanol is evaporated therefrom and the residue is diluted with water and acidified with hydrochloric acid.
The solution is extracted with ethyl acetate and the extract is washed with water, dried, condensed, and further triturated with n-hexane to obtain 13.6 g of 2-[4-{(RS)-2-(p-chlorobenzenesulfonylamino)-propyl}phenoxy]-2-methylpropionic acid as colorless prisms.
M. P. : 136-137 °C
IR (Nujol) $cm^{-1}$ : 1710
Mass (m/z) : 411 $(M^+)$
NMR ($CDCl_3$) δ : 1.13 (d., J=6.5Hz, 3H), 1.60(s., 6H)
(3) The above-obtained product is treated in the same manner as described in Example 1-(3) and the eluate is lyophilized to obtain 1.98 g of sodium 2-[4-{(RS)-2-(p-chlorobenzenesulfonylamino) propyl}phenoxy]-2-methylpropionate as colorless powder. Yield : 82%
IR (Nujol) $cm^{-1}$ : 1590
Mass (m/z) : 456 $(M+Na)^+$, 434$(M+H)^+$
NMR (DMSO-$d_6$) δ : 0.87 (d., J=6.3Hz, 3H), 1.36(s., 6H)

Example 4

(1) 2.28 g of ethyl 2-[4-{(RS)-2-benzenesufonylaminopropyl} phenoxy]-2-methylpropionate, 1.6 g of methyl iodide and 4.6 g of a mixture of potassium fluoride, aluminum oxide and water are added to 10 ml of acetonitrile, and the mixture is stirred at room temperature for 1.5 hours. The insoluble substance is filtered off and the filtrate is diluted with ethyl acetate, washed, dried, and evaporated to remove the solvent to obtain 2.32 g of ethyl 2-[4-{(RS)-2-(N-benzenesulfonyl-N-methylamino)propyl}phenoxy]-2-methylpropionate as an oil.
IR (Liquid) $cm^{-1}$ : 1730
Mass (m/z) : 419 $(M^+)$
NMR ($CDCl_3$) δ : 0.93 (d., J=6.8Hz, 3H), 1.25 ( t., J=7.3Hz, 3H), 1.58 (s., 6H), 2.73 (s., 3H), 4.23 (d., J=7.3Hz, 2H)
(2) 2.22 g of the above-obtained product is dissolved in 6 ml of ethanol, and a solution of one g of sodium hydroxide in 2 ml of water is added thereto, and the mixture is stirred at room temperature for 5 hours. Then, the solution is condensed and the residue is acidified with 10% hydrochloric acid and extracted with ethyl acetate. The extract is washed with water, dried and evaporated to remove the solvent to obtain 2.15 g of 2-[4-{(RS)-2-(N-benzenesulfonyl-N-methylamino)propyl}phenoxy]-2-methylpropionic acid as an oil.
IR (Liquid) $cm^{-1}$ : 1740
Mass (m/z) : 391 $(M^+)$
NMR ($CDCl_3$) δ : 0.94 (d., J=6.8Hz, 3H), 1.58(s., 6H), 2.74(s., 3H)
(3) 2.15 g of the above-obtained product are treated in the same manner as described in Example 1-(3) and the resulting product is lyophilized to obtain 1.5 g of sodium 2-[4-{(RS)-2-(N-benzenesulfonyl-N-methylamino)propyl}phenoxy]-2-methylpropionate as colorless powder.
IR (Nujol) $cm^{-1}$ : 1600

Mass (m/z) : 436 $(M+Na)^+$, 414$(M+H)^+$
NMR (DMSO-$d_6$) $\delta$ : 0.82 (d., J=6.8Hz, 3H), 1.35(s., 6H), 2.67(s., 3H)

Examples 5 to 25

The corresponding starting compounds are treated in the same manner as described in Examples 1 to 4 to obtain the compounds listed in Tables 2 to 6.

Table 2

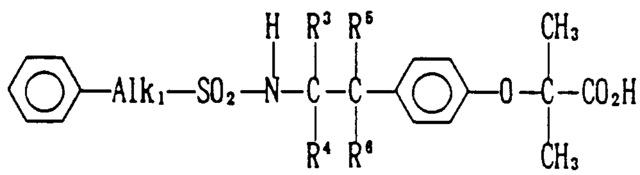

| Ex. Nos. | $Alk_1$ | $R^3$ | $R^4$ | $R^5$ | $R^6$ | Physicochemical Properties |
|---|---|---|---|---|---|---|
| 5 | — | H | H | $-CH_3$ | H | Free Acid : Colorless Needles<br>M. P. 152-154°C<br>(Ethyl acetate-n-Hexane)<br>Sodium Salt : Powder<br>$IR^{Nujol}$ $\nu_{Max}$ $(cm^{-1})$ : 1590<br>Mass (m/z) : 422 $(M+Na)^+$ |
| 6 | — | H | H | $-CH_3$ | $-CH_3$ | Free Acid : Colorless Needles<br>M. P. 166-168°C<br>(Ethyl acetate-n-Hexane)<br>Sodium Salt : Colorless Needles<br>M. P. 171-174°C(Ethanol-ether)<br>$IR^{Nujol}$ $\nu_{Max}$ $(cm^{-1})$ : 1590<br>Mass (m/z) : 436 $(M+Na)^+$ |
| 7 | — | $-CH_3$ | $-CH_3$ | H | H | Free Acid : Colorless Prisms<br>M. P. 145.5-146.5°C<br>(Ethyl acetate-n-Hexane)<br>Sodium Salt : Powder<br>$IR^{Nujol}$ $\nu_{Max}$ $(cm^{-1})$ : 1590<br>Mass (m/z) : 436 $(M+Na)^+$ |
| 8 | $-CH_2$ | $-CH_3$ | H | H | H | Free Acid : Oil<br>$IR^{Nujol}$ $\nu_{Max}$ $(cm^{-1})$ : 1720<br>Mass (m/z) : 391 $(M^+)$<br>Sodium Salt : Powder<br>$IR^{Nujol}$ $\nu_{Max}$ $(cm^{-1})$ : 1600<br>Mass (m/z) : 436 $(M+Na)^+$ |

All the compounds of Ex. Nos. 5 to 8 are racemates.

## Table 3

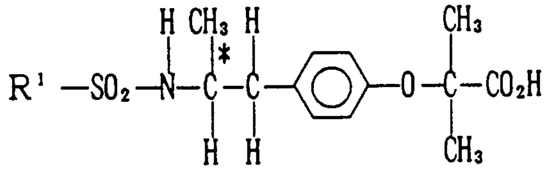

| Ex. Nos. | $R^1$ | Absolute Configuration | Physicochemical Properties |
|---|---|---|---|
| 9 | 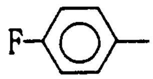 | RS | Free Acid : Oil<br>$IR^{Liquid}$ $\nu_{Max}$ $(cm^{-1})$ : 1720<br>Mass (m/z) : 395 $(M^+)$<br>Sodium Salt : Powder<br>$IR^{Nujol}$ $\nu_{Max}$ $(cm^{-1})$ : 1590<br>Mass (m/z) : 440 $(M+Na)^+$ |
| 10 | 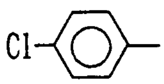 | R | Free Acid : Oil<br>$[\alpha]_D$ : +16.2°<br>(C=1.62, $CHCl_3$, 20 °C)<br>$IR^{Liquid}$ $\nu_{Max}$ $(cm^{-1})$ : 1720<br>Sodium Salt : Powder<br>$[\alpha]_D$ : −3.77°<br>(C=1.22, $CH_3OH$, 20°C) |
| 11 | 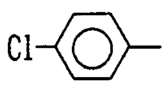 | S | Free Acid : Oil<br>$[\alpha]_D$ : −15.1°<br>(C=0.37, $CHCl_3$, 20 °C)<br>$IR^{Liquid}$ $\nu_{Max}$ $(cm^{-1})$ : 1710<br>Sodium Salt : Powder<br>$[\alpha]_D$ : +4.07°<br>(C=1.03, $CH_3OH$, 20°C) |
| 12 | 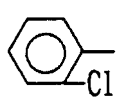 | RS | Free Acid : Oil<br>$IR^{Liquid}$ $\nu_{Max}$ $(cm^{-1})$ : 1740<br>Mass (m/z) : 411 $(M^+)$<br>Sodium Salt : Powder<br>$IR^{Nujol}$ $\nu_{Max}$ $(cm^{-1})$ : 1595<br>Mass (m/z) : 456 $(M+Na)^+$ |

# Table 4

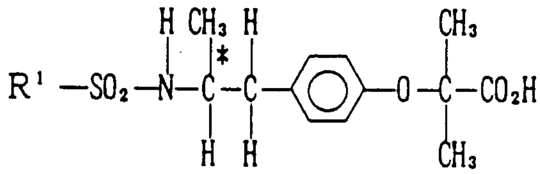

| Ex. Nos. | $R^1$ | Absolute Configuration | Physicochemical Properties |
|---|---|---|---|
| 13 | 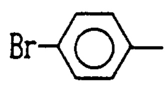 | RS | Free Acid : Needles<br>M. P. 155−156℃<br>(Ethyl acetate−n-Hexane) |
| | | | Sodium Salt : Powder<br>$IR^{Nujol}$ $\nu_{Max}$ ($cm^{-1}$) : 1595<br>Mass (m/z) : 479 $(M+H)^+$ |
| 14 | 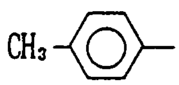 | RS | Free Acid : Oil<br>$IR^{Liquid}$ $\nu_{Max}$ ($cm^{-1}$) : 1720<br>Mass (m/z) : 391 ($M^+$) |
| | | | Sodium Salt : Powder<br>$IR^{Nujol}$ $\nu_{Max}$ ($cm^{-1}$) : 1600<br>Mass (m/z) : 436 $(M+Na)^+$ |
| 15 | 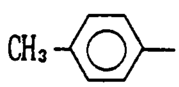 | R | Free Acid : Oil<br>$[\alpha]_D$ : +14.9°<br>(C=2.34, $CHCl_3$, 20℃) |
| | | | Sodium Salt : Colorless Needles<br>M. P. 66−68℃<br>$[\alpha]_D$ : −6.46°<br>(C=2.94, $CH_3OH$, 20℃) |
| 16 | 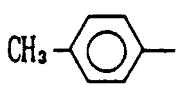 | S | Free Acid : Oil<br>$[\alpha]_D$ : −14.2°<br>(C=2.74, $CHCl_3$, 20℃) |
| | | | Sodium Salt : Colorless Needles<br>M. P. 70−74℃<br>$[\alpha]_D$ : +7.14°<br>(C=2.38, $CH_3OH$, 20℃) |

Table 5

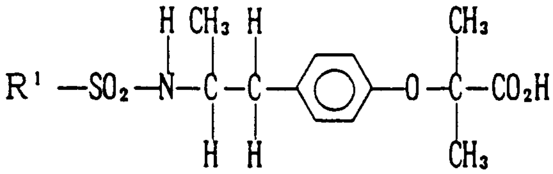

| Ex. Nos. | $R^1$ | Physicochemical Properties |
|---|---|---|
| 17 | $CH_3O$ 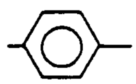 | Free Acid : Oil<br>$IR^{Liquid}$ $\nu_{Max}$ ($cm^{-1}$) : 1720<br>Mass (m/z) : 407 ($M^+$) |
| | | Sodium Salt : Powder<br>$IR^{Nujol}$ $\nu_{Max}$ ($cm^{-1}$) : 1600<br>Mass (m/z) : 452 $(M+Na)^+$ |
| 18 | AcNH 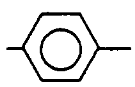 | Free Acid : Needles<br>M. P. 172-173℃<br>(Ethyl acetate-n-Hexane) |
| | | Sodium Salt : Powder<br>$IR^{Nujol}$ $\nu_{Max}$ ($cm^{-1}$) : 1680, 1595<br>Mass (m/z) : 479 $(M+Na)^+$ |
| 19 | 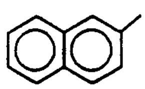 | Free Acid : Needles<br>M. P. 123-125℃<br>(Ethyl acetate-n-Hexane) |
| | | Sodium Salt : Powder<br>$IR^{Nujol}$ $\nu_{Max}$ ($cm^{-1}$) : 1595<br>Mass (m/z) : 472 $(M+Na)^+$ |
| 20 | 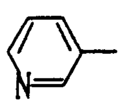 | Sodium Salt : Powder<br>$IR^{Nujol}$ $\nu_{Max}$ ($cm^{-1}$) : 1580<br>Mass (m/z) : 423 $(M+Na)^+$ |

All the compounds of Ex. Nos. 17 to 20 are racemates.

Table 6

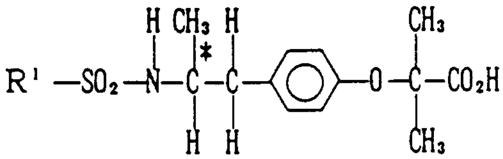

| Ex. Nos. | $R^1$ | Absolute Configuration | Physicochemical Properties |
|---|---|---|---|
| 21 | 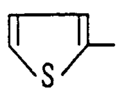 | RS | Free Acid : Oil<br>$IR^{Liquid}$ $\nu_{Max}$ ($cm^{-1}$) : 1720<br>Mass (m/z) : 383 ($M^+$) |
| | | | Sodium Salt : Colorless Needles<br>M. P. >110℃ (dec.)<br>$IR^{Nujol}$ $\nu_{Max}$ ($cm^{-1}$) : 1590<br>Mass (m/z) : 428 $(M+Na)^+$ |
| 22 | 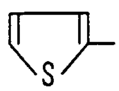 | R | Free Acid : Oil<br>$[\alpha]_D$ : +6.53°<br>(C=1.04, $CHCl_3$, 20℃) |
| | | | Sodium Salt : Powder<br>$[\alpha]_D$ : −7.04°<br>(C=1.06, $CH_3OH$, 20℃) |
| 23 | 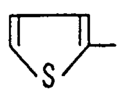 | S | Free Acid : Oil<br>$[\alpha]_D$ : −4.31°<br>(C=1.02, $CHCl_3$, 20℃) |
| | | | Sodium Salt : Powder<br>$[\alpha]_D$ : +7.40°<br>(C=1.00, $CH_3OH$, 20℃) |
| 24 | $CH_3-$ | RS | Free Acid : Oil<br>$IR^{Liquid}$ $\nu_{Max}$ ($cm^{-1}$) : 1740<br>Mass (m/z) : 315 ($M^+$) |
| | | | Sodium Salt : Powder<br>$IR^{Nujol}$ $\nu_{Max}$ ($cm^{-1}$) : 1600<br>Mass (m/z) : 360 $(M+Na)^+$ |
| 25 | $CH_3(CH_2)_3-$ | RS | Free Acid : Oil<br>$IR^{Liquid}$ $\nu_{Max}$ ($cm^{-1}$) : 1720<br>Mass (m/z) : 357 ($M^+$) |
| | | | Sodium Salt : Powder<br>$IR^{Nujol}$ $\nu_{Max}$ ($cm^{-1}$) : 1600<br>Mass (m/z) : 402 $(M+Na)^+$ |

Example 26

3.0 g of sodium 2-[4-{(R)-2-(p-chlorobenzenesufonylamino) propyl} phenoxy]-2-methylpropionate are dissolved in 20 ml of thionyl chloride and the reaction mixture is stirred at room temperature for 2 hours. Excessive thionyl chloride is removed from the reaction mixture under reduced pressure. The residue ( 2-[4-{(R)-2-(p-chlorobenzenesufonylamino) propyl}phenoxy]-2-methylpropionyl chloride ) is dissolved in 10 ml of chloroform, and the solution is added dropwise to a mixture of 10 ml of 50% aqueous dimethylamine solution and 20 ml of chloroform. The mixture is stirred at room temperature for 2 hours and the organic layer is separated therefrom. This organic layer is washed with water, dried, and evaporated under reduced pressure to remove the solvent. The residue is purified by silica gel column chromatography (solvent : ethyl acetate/ n-hexane = 1 : 1) to obtain 2.52 g of N,N-dimethy-2-[4-{(R)-2-(p-chlorobenzenesulfonylamino)-propyl}phenoxy]-2-methylpropanamide. Yield : 83%
M. P.: 107-108 ° C (Recrystallized from a mixture of isopropyl alcohol and isopropyl ether)
IR (Nujol) $cm^{-1}$ : 3190, 1625, 1580
Mass (m/z) : 439 ($M^+$)
NMR ($CDCl_3$) $\delta$ : 1.10 (d., J=6.8Hz, 3H), 1.62 (s., 6H), 2.60 ( d., J=6.8Hz, 2H), 2.96 and 3.17 (d., 6H), 3.40-3.60 (m., 1H), 4.31 (d., J=7.3Hz, 1H), 6.69 (d., 2H), 6.88 (d., 2H), 7.42 (d., 2H), 7.68 (d., 2H),

Example 27

(1) 2-[4-{(R)-2-(p-chlorobenzenesufonylamino)propyl}phenoxy]-2-methylpropionoyl chloride is prepared from 3.0 g of sodium 2-[4-{(R)-2-(p-chlorobenzenesufonylamino)propyl}phenoxy]-2-methylpropionate and 20 ml of thionyl chloride. The product and 1.4 g of methyl $\beta$-aminopropionate hydrochloride are treated in the same manner as described in Example 26, except that the reaction is carried out in two-phase solvent (ie., aqueous sodium bicarbonate solution and chloroform) to obtain 2.92 g of N-methoxycarbonylethyl-2-[4-{(R)-2-(p-chlorobenzenesulfonylamino) propyl}phenoxy]-2-methylpropanamide.
Yield : 85%
IR (Nujol) $cm^{-1}$ : 3400, 3280, 3200, 1730, 1660
Mass (m/z) : 497$(M+H)^+$
NMR ($CDCl_3$) $\delta$ : 1.11 (d., J=6.3Hz, 3H), 1.49(s., 6H), 2.56(d., J=6.3Hz, 2H), 2.64(J=6.4Hz, 2H), 3.40-3.70 (m., 3H), 3.63(s., 3H), 4.41 (d., J=7.8Hz, 1H), 6.76 (d., 2H), 6.92(d, 2H), 7.17(d., 1H), 7.42(d., 2H), 7.68(d., 2H)
(2) 2.1 g of the above-obtained product, 2 ml of 4N aqueous sodium hydroxide solution and 20 ml of methanol are stirred at room temperature for 2 hours. Methanol are removed from the reaction mixture under reduced pressure. The residue is dissolved in 100 ml of water and ethyl acetate is added thereto, and the mixture is shaken. The aqueous layer is separated from the mixture and 10% hydrochloric acid is added thereto and extracted with ethyl acetate. The extract is washed, dried, and evaporated to remove the solvent to obtain 1.95 g of N-carboxyethyl-2-[4-{(R)-2-(p-chlorobenzenesulfonylamino) propyl}phenoxy]-2-methylpropanamide.
IR (Liquid) $cm^{-1}$ : 3400, 3280, 1730, 1660
Mass (m/z) : 483 $(M+H)^+$
NMR ($CDCl_3$) $\delta$ : 1.03 (d., J=6.4Hz, 3H), 1.56 and 1.58 (d., 6H) 2.40-2.88 (m., 4H), 3.40-3.70 ( m., 3H), 5.02 (d., J=7.8Hz, 1H), 6.72 (d., J=8.8Hz, 2H), 6.96 (d., J=8.8.Hz, 2H), 7.43 (d, J=8.8Hz, 2H), 7.70(d., J=8.8Hz, 2H)
(3) The above-obtained product is dissolved in one ml of 4N aqueous sodium hydroxide solution and the solution is treated in the same manner as described in Example 1-(3) to obtain 1.80 g of N-carboxyethyl-2-[4-{(R)-2-(p-chlorobenzenesulfonylamino) propyl}phenoxy]-2-methylpropanamide sodium salt.
Yield : 85%
IR (Nujol) $cm^{-1}$ : 3300, 3280, 1660, 1580
Mass (m/z) : 527 $(M+Na)^+$, 505$(M+H)^+$
NMR ($D_2O$) $\delta$ : 1.17 (d., J=6.8Hz, 3H), 1.47(s., 6H), 2.42 (t., J=7.3Hz, 2H), 2.51 (dd., J=14.2Hz, 9.3Hz, 1H), 2.72 (dd., J=13.7.Hz, 4.9Hz, 1H), 3.48(t, J=7.3Hz, 2H), 3.40-3.60(m., 1H), 6.74(d., J=8.3Hz, 2H), 6.97 (d., J=8.3Hz, 2H), 7.47(d., J=8.8Hz, 2H), 7.58(d., J=8.8Hz, 2H)

Example 28

(1) 2-[4-{(R)-2-(p-methylbenzenesufonylamino)propyl} phenoxy]-2-methylpropionoyl chloride is prepared from 3.01 g of sodium 2-[4-{(R)-2-(p-methylbenzenesufonylamino)propyl) phenoxy]-2-methylpropionate and 20 ml of thionyl chloride. The product and 0.975 g of methyl $\gamma$-aminobutanoate hydrochloride are treated in the same manner as described in Example 26 to obtain 2.4 g of N-methoxycarbonylpropyl-2-[4-{(R)-2-(p-methylbenzenesulfonylamino)propyl}phenoxy]-2-methylpropanamide. Yield : 85%
IR (Liquid) $cm^{-1}$ : 3380, 3280, 3200, 1740, 1660

Mass (m/z) : 491$(M+H)^+$
NMR ($CDCl_3$) δ : 1.04 (d., J=6.6Hz, 3H), 1.48(s., 6H), 1.65-2.05(m., 2H), 2.33(t., J=6.8Hz, 2H), 2.42(s., 3H) 2.56-2.70 (m., 2H), 3.20-3.60(m., 3H), 3.65(s., 3H), 4.48 (d., J=7.5Hz, 1H), 6.70-7.00 (br., 1H), 6.75(d, J=8.7Hz, 2H), 6.96(d., J=8.7Hz, 2H), 7.25(d., J=9Hz, 2H), 7.70(d., J=9Hz, 2H)

(2) The above-obtained product is treated in the same manner as described in Example 27-(2) and (3) to obtain 1.7 g of N-carboxypropyl-2-[4-{(R)-2-(p-methylbenzenesulfonylamino) propyl}phenoxy]-2-methylpropanamide sodium salt.
Yield : 86%
IR (Nujol) $cm^{-1}$ : 3300, 3280, 1660, 1580
Mass (m/z) : 499 $(M+Na)^+$ ,477$(M+H)^+$
NMR ($D_2O$) δ : 1.06 (d., J=6.4Hz, 3H), 1.48(s., 6H), 1.64-1.68(m., 2H), 2.14(t., J=7.3Hz, 2H), 2.40(s., 3H), 2.44-2.72 (m., 2H), 3.25(t., J=7.3Hz, 2H), 3.32-3.52(m., 1H), 6.75 (d., J=8.7Hz, 2H), 6.97(d, J=8.7Hz, 2H), 7.32(d., J=8.8Hz, 2H), 7.57(d., J=8.8Hz, 2H)

Reference Example 1

(1) 29.76 g of 4-{(RS)-2-aminopropyl}phenol hydrobromide and 53 g of potassium carbonate are added in a mixture of 400 ml of ethyl acetate and 170 ml of water, and further 21.11 g of carbobenzoxy chloride are added dropwise thereto. The reaction mixture is stirred at room temperature for 40 minutes.

The organic layer is separated therefrom, washed, dried and evaporated to remove the solvent to obtain 37.5 g of 4-{(RS)-2-(carbobenzoxyamino)propyl}phenol as an oil.

(2) 12.20 g of the above-obtained product, 23.5 g of ethyl 2-bromo-2-methylpropionate and 16.6 g of potassium carbonate are added to 180 ml of acetone and the mixture is refluxed for 6 hours. Further, 11.8 g of ethyl 2-bromo-2-methylpropionate are added thereto and the mixture is refluxed for 24 hours. Inorganic substances are removed from the reaction mixture by filtration, and the filtrate is condensed. The residue is dissolved in ethyl acetate, washed with water, dried, and then the solvent is evaporated therefrom. The residue is dissolved in 150 ml of ethanol and 1.7 g of 10% palladium-carbon are added therein and catalytic reduction is carried out under atmospheric pressure in hydrogen atmosphere. The palladium-carbon is filtered off and the filtrate is condensed.

The residue is dissolved in ethyl acetate and extracted with 10% hydrochloric acid. The hydrochloric acid layer is alkalized with potassium carbonate and extracted with ethyl acetate. The extract is dried and evaporated to remove the solvent. Further, the residue is dissolved in ethanol and a mixture of 8.0 g of oxalic acid and ether are added thereto. The crystalline precipitates are filtered to obtain 11.0 g of ethyl 2-[4-{(RS)-2-aminopropyl}phenoxy]-2-methylpropionate oxalate as colorless needles. Yield : 71%
M. P. : 115-117 °C
IR (Nujol) $cm^{-1}$ : 1730,
Mass (m/z) : 265$(M^+)$
NMR (DMSO-$D_6$) δ : 1.06(d., J=6.3Hz, 3H), 1.16(t., J=7.3Hz, 3H), 1.50(s., 6H)

Reference Example 2

(1) 4-{(R)-2-aminopropyl}phenol hydrobromide is treated in the same manner as described in Reference Example 1 to obtain ethyl 2-[4-{(R)-2-aminopropyl}phenoxy]-2-methylpropionate oxalate as colorless needles. Yield : 84%
M. P. : 125-126 °C (Recrystallized from a mixture of ethanol and ether)
$[\alpha]_D^{20}$ : -5.87° (c=1.02, methanol)

Reference Example 3

A mixture of 3.06 g of 4-(2-methyl-2-aminopropyl)phenol oxalate, 40 ml of 1,2-dichloroethane, 8.48 g of benzenesulfonyl chloride and 9.69 g of triethylamine is refluxed for 5 hours. The solvent is removed therefrom and a mixture of 50 ml of methanol and 50 ml of 10% aqueous sodium hydroxide solution are added thereto, and the mixture is refluxed for 40 minutes. Methanol is removed therefrom by evaporation and the residue is adjusted to pH one with 6N hydrochloric acid and the solution is extracted with ethyl acetate. The extract is washed with water and 5% aqueous sodium bicarbonate, further washed with water, dried, and then the solvent is evaporated therefrom. The resulting oily substance is purified by silica gel column chromatography (solvent : ether/ n-hexane = 3 : 2) and recrystallized from a mixture of ethyl acetate and n-hexane to obtain 2.0 g of 4-{2-methyl-2-(benzenesulfonylamino)propyl}phenol as colorless

prisms.
Yield : 55%
M. P. : 147-148 °C
Mass (m/z) : 305 ($M^+$)

Reference Example 4

6.0 g of 4-{(S)-2-aminopropyl}phenol hydrobromide are added to a solution of 10.1 g of sodium bicarbonate in a mixture of 80 ml of ethyl acetate and 40 ml of water. 20 ml of ethyl acetate containing 8.2 g of p-chlorobensenesulfonyl chloride are added dropwise to the mixture for 10 minutes. The mixture is stirred at room temperature for 2 hours and acidified with 10% hydrochloric acid. The organic layer is separated therefrom and the water layer is extracted with ethyl acetate. The extract and the organic layer are combined, washed with water, dried, and evaporated to remove the solvent under reduced pressure. The residue is triturated with n-hexane and recrystallized from a mixture of ethyl acetate and n-hexane to obtain 9.5 g of (S)-4-{2-(p-chlorobenzenesulfonylamino)propyl}phenol as colorless needles. Yield : 73%
M. P. : 103-105 °C (Recrystallized from a mixture of ethyl acetate and n-hexane)
IR (Nujol) $cm^{-1}$ : 3300
Mass (m/z) : 325($M^+$)
$[\alpha]_D^{20}$ : +2.91° ( c=1.03, methanol)

Reference Example 5

(1) P-methylbenzenesulfonyl chloride is treated in the same manner as described in Reference Example 4 to obtain 4-{(S)-2-(p-methylbenzenesulfonylamino)propyl}phenol as colorless prisms.
Yield : 86%
M. P. : 119-120.5°C (Recrystallized from a mixture of ethyl acetate, isopropyl ether and n-hexane)
$[\alpha]_D^{20}$ : -4.71° (c=1.91, chloroform)

Reference Example 6

2.4 g of 2-thiophenesulfonyl chloride are added to a solution of 3.1 g of 4-{(S)-2-aminopropyl}phenol hydrobromide and 3.8 g of triethylamine in 30 ml of tetrahydrofuran. The reaction mixture is stirred at room temperature for 3 hours, diluted with water and extracted with ethyl acetate. The organic layer is washed with water, dried and evaporated to remove the solvent to obtain quantitatively 4.09 g of 4-{(S)-2-(2-thienylsulfonylamino) propyl}phenol as a pale yellow oil.
IR (Liquid) $cm^{-1}$ : 3430, 3300
Mass (m/z) : 297($M^+$)
$[\alpha]_D^{20}$ : +5.76° (c=1.04, chloroform)

## Claims

**1.** A phenoxyalkanoic acid compound of the formula [I]:

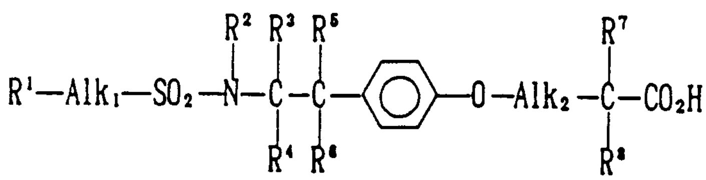

[I]

wherein $R^1$ is a substituted or unsubstituted phenyl group, naphthyl group, a sulfur- or nitrogen-containing 5- or 6-membered heterocyclic group or a lower alkyl group; $R^2$ is hydrogen atom or a lower alkyl group; one or two of $R^3$ - $R^6$ is (are) lower alkyl group(s) and others are hydrogen atoms; each of $R^7$ and $R^8$ is a lower alkyl group; and each of $Alk_1$ and $Alk_2$ is single bond or a lower alkylene group, or a pharmaceutically acceptable amide or salt thereof.

**2.** The compound according to claim 1, wherein $R^1$ is phenyl group, a halogenophenyl group, a lower

alkylphenyl group, a lower alkoxyphenyl group, a lower alkanoylaminophenyl group, naphthyl group, pyridyl group, thienyl group or a lower alkyl group.

**3.** The compound according to claim 2, wherein $R^1$ is phenyl group, a halogenophenyl group, a lower alkylphenyl group, a lower alkoxyphenyl group or thienyl group.

**4.** The compound according to claim 3, wherein $R^1$ is a halogenophenyl group, a lower alkylphenyl group or thienyl group; each of $R^2$ and $R^4$-$R^6$ is hydrogen atom; each of $R^3$, $R^7$ and $R^8$ is a lower alkyl group; and each of $Alk_1$ and $Alk_2$ is single bond.

**5.** 2-[4-{2-(p-chlorobenzenesulfonylamino)propyl}phenoxy]-2-methylpropionic acid or a pharmaceutically acceptable salt thereof.

**6.** 2-[4-{2-(p-methylbenzenesulfonylamino)propyl}phenoxy]-2-methylpropionic acid or a pharmaceutically acceptable salt thereof.

**7.** 2-[4-{2-(2-thienylsulfonylamino)propyl}phenoxy]-2-methylpropionic acid or a pharmaceutically acceptable salt thereof.

**8.** A pharmaceutical composition which comprises a therapeutically effective amount of the compound as set forth in claim 1 in admixture with a conventional pharmaceutically acceptable carrier or diluent.

**9.** A process for preparing a phenoxyalkanoic acid compound of the formula [I]:

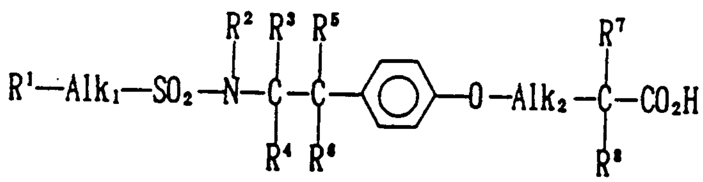
[I]

wherein $R^1$ is a substituted or unsubstituted phenyl group, naphthyl group, a sulfur- or nitrogen-containing 5- or 6-membered heterocyclic group or a lower alkyl group; $R^2$ is hydrogen atom or a lower alkyl group; one or two of $R^3$ - $R^6$ is (are) lower alkyl group(s) and others are hydrogen atoms; each of $R^7$ and $R^8$ is a lower alkyl group; and each of $Alk_1$ and $Alk_2$ is single bond or a lower alkylene group, or a pharmaceutically acceptable amide or salt thereof,

which comprises condensing an amine compound of the formula [II]:

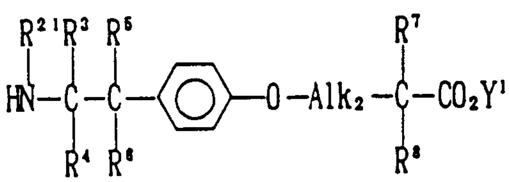
[II]

wherein $R^{21}$ is hydrogen atom or a lower alkyl group; $-CO_2Y^1$ is carboxyl group or a protected carboxyl group; and other symbols are the same as defined above, or a salt thereof with a sulfonic acid compound of the formula [III]:

$R^1$-$Alk_1$-$SO_2$ -$X^1$ [III]

wherein $X^1$ is a reactive residue or hydroxy group and $R^1$ and $Alk_1$ are the same as defined above, or a salt thereof,

(a) when $-CO_2Y^1$ is a protected carboxyl group, removing the protecting group therefrom, and
(b) when $R^{21}$ is hydrogen atom, optionally subjecting the product to N-alkylation, and

(c) if required, further converting the product into a pharmaceutically acceptable amide or salt thereof.

**10.** A process for preparing a phenoxyalkanoic acid compound of the formula [I]:

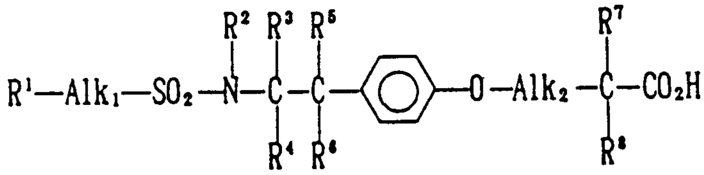

[I]

wherein $R^1$ is a substituted or unsubstituted phenyl group, naphthyl group, a sulfur- or nitrogen-containing 5- or 6-membered heterocyclic group or a lower alkyl group; $R^2$ is hydrogen atom or a lower alkyl group; one or two of $R^3$ - $R^6$ is (are) lower alkyl group(s) and others are hydrogen atoms; each of $R^7$ and $R^8$ is a lower alkyl group; and each of $Alk_1$ and $Alk_2$ is single bond or a lower alkylene group, or a pharmaceutically acceptable amide or salt thereof,

which comprises condensing a phenol compound of the formula [IV]:

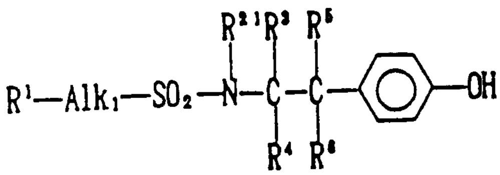

[IV]

wherein $R^{21}$ is hydrogen atom or a lower alkyl group and other symbols are the same as defined above, or a salt thereof with an acetic acid compound of the formula [V]:

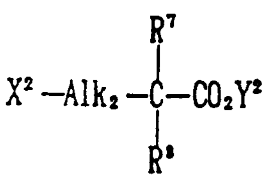

[V]

wherein $X^2$ is a reactive residue or hydroxy group, $-CO_2Y^2$ is carboxyl group or a protected carboxyl group and other symbols are the same as defined above, or a salt thereof,

(a) when $-CO_2Y^2$ is a protected carboxyl group, removing the protecting group therefrom, and

(b) when $R^{21}$ is hydrogen atom, optionally subjecting the product to N-alkylation, and

(c) if required, further converting the product into a pharmaceutically acceptable amide or salt thereof.

European Patent Office

# EUROPEAN SEARCH REPORT

Application Number

EP 92 10 3682

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 004 011 (BOEHRINGER MANNHEIM)<br>& JP-A-54 122 250 (BOEHRINGER MANNHEIM)<br>--- | | C07C311/17<br>C07C311/13<br>C07C311/29<br>C07C311/46<br>C07C311/04<br>C07D213/71<br>C07D333/34<br>A61K31/19<br>A61K31/38<br>A61K31/44 |
| D,A | EP-A-0 255 728 (TANABE SEIYAKU)<br>& JP-A-64 000 062 (TANABE SEIYAKU)<br>--- | | |
| A | EP-A-0 239 907 (BOEHRINGER MANNHEIM)<br>----- | | |
| | | | TECHNICAL FIELDS SEARCHED (Int. Cl.5) |
| | | | C07C<br>C07D |

The present search report has been drawn up for all claims

| Place of search | Date of completion of the search | Examiner |
|---|---|---|
| THE HAGUE | 16 JUNE 1992 | ZAROKOSTAS K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)